# EUROPEAN PATENT APPLICATION

(11) **EP 4 459 334 A1**
(43) Date of publication of application: **06.11.2024**
(21) Application number: 22914868.9
(22) Date of filing: 28.12.2022
(51) Int. Cl.: G01V 5/00, G01N 23/046, A61B 6/03

(54) **SCANNING INSPECTION DEVICE**

(30) Priority: 30.12.2021 CN 202111668982
(71) Applicant: Nuctech Company Limited, TongFang Building Shuangqinglu Haidian District Beijing 100084 (CN)
(72) Inventor: ZONG, Chunguang, Beijing 100084 (CN); YU, Hao, Beijing 100084 (CN); SONG, Quanwei, Beijing 100084 (CN); LIU, Bicheng, Beijing 100084 (CN); YIN, Wei, Beijing 100084 (CN); WANG, Weizhen, Beijing 100084 (CN); SUN, Shangmin, Beijing 100084 (CN); HU, Yu, Beijing 100084 (CN)
(74) Representative: Gunzelmann, Rainer
(86) International application number: PCT/CN2022/142641
(87) International publication number: WO 2023/125616

(57) **Abstract**

The present disclosure relates to a scanning inspection device, comprises a first rotating frame (1), a first drive device, a ray source (2), a detector (3), a cooling device for cooling the ray source (2), and a second drive device, wherein the center of the first rotating frame (1) is provided with a conveying channel for passage of an object to be inspected (6); the first drive device in drive connection with the first rotating frame (1) to drive the first rotating frame (1) to rotate, wherein the ray source (2), the detector (3), and the cooling device for cooling the ray source (2) are each mounted on the first rotating frame (1) and rotate with the first rotating frame (1); and a second drive device configured to drive the cooling device to rotate toward a direction opposite to a rotation direction of the first rotating frame (1), so that the cooling device is maintained in a fixed attitude during rotation with the first rotating frame

## Description

### Cross-Reference to Related Applications

This disclosure is based on and claims priority to Chinese Patent Application No. 202111668982.8, entitled "Scanning Inspection Device", filed on December 30, 2021, which is hereby incorporated by reference in its entirety.

### Field of the Invention

The present disclosure relates to the technical field of security inspection, in particular to a scanning inspection device.

### Background of the Invention

In the field of container security inspection, most inspection equipment used is single-view or dual-view transmissive equipment. A system uses an intensity change of a high-energy X-ray generated by a ray source after passing through an object to be inspected to reflect the distribution of density of the object to be inspected, and converts the intensity change of the ray into an image gray scale to obtain a transmission image. However, when goods with complex texture in a container are placed in a superimposed manner, it is very difficult to determine a hidden object in the container based on a transmission image from one or two view angles, and the accuracy is not high.

In order to solve the above problem, there is a container inspection system with a CT (Computed Tomography) scanning function in the related art. The system can perform tomography scanning on a suspicious part of an object to be inspected and reconstruct a two-dimensional image of an inspected cross-section. This type of inspection system can implement continuous rotational scanning, and thus can reconstruct an object to be inspected in three dimensions and provide more information. However, during container inspection, as a ray source has high energy and generates a large amount of heat, it is liable to cause damage to the ray source itself and neighboring components, and result in failure of the ray source and the neighboring components, which affects normal working of the inspection system. How to cool the ray source has become a major problem to be solved urgently.

It is to be noted that the information disclosed in the background section is only intended to enhance understanding of the general background of the present disclosure, and should not be deemed as an admission or any form of implication that the information constitutes the prior art well known to those skilled in the art.

### Summary of the Invention

Embodiments of the present disclosure provide a scanning inspection device capable of cooling a ray source in a timely and effective manner.

According to an aspect of the present disclosure, a scanning inspection device is provided, which includes:
a first rotating frame, with a conveying channel for passage of an object to be inspected being provided in the center of the first rotating frame;
a first drive device in drive connection with the first rotating frame to drive the first rotating frame to rotate,
wherein a ray source, a detector, and a cooling device for cooling the ray source are each mounted on the first rotating frame and rotate with the first rotating frame; and
a second drive device configured to drive the cooling device to rotate toward a direction opposite to a rotation direction of the first rotating frame, so that the cooling device is maintained in a fixed attitude during rotation with the first rotating frame.

In some embodiments, the cooling device is disposed outside a scanning range of the ray source.

In some embodiments, the scanning inspection device further includes a second rotating frame, the second rotating frame being mounted on the first rotating frame, the cooling device being mounted on the second rotating frame, and the second drive device being in drive connection with the second rotating frame to drive the second rotating frame to rotate relative to the first rotating frame.

In some embodiments, the cooling device includes a refrigerating device and a first conveying pipe, the first conveying pipe being in communication with the refrigerating device and configured to convey a heat exchange medium from the refrigerating device to the ray source for cooling thereof.

In some embodiments, the cooling device further includes a second conveying pipe, the second conveying pipe being connected between an outlet of the first conveying pipe and the refrigerating device to return the heat exchange medium after heat exchange with the ray source to the refrigerating device.

In some embodiments, the scanning inspection device further includes a rotary joint, which includes a first stator and a first rotor rotationally connected to the first stator, the first stator being connected to the first rotating frame, and the first rotor being connected to the second rotating frame; and the first conveying pipe includes a first pipe section and a second pipe section, the first pipe section being connected between the refrigeration device and the first stator, and the second pipe section being connected to the first rotor, and the first pipe section and the second pipe section being in fluid communication through a first fluid pass age between the first stator and the first rotor.

In some embodiments, the scanning inspection device further includes a rotary joint, which includes a first stator and a first rotor rotationally connected to the first stator, the first stator being connected to the first rotating frame, and the first rotor being connected to the second rotating frame; the first conveying pipe includes a first pipe section and a second pipe section, the first pipe section being connected between the refrigeration device and the first stator, and the second pipe section being connected to the first rotor, and the first pipe section and the second pipe section being in fluid communication through a first fluid passage between the first stator and the first rotor; and the second conveying pipe includes a third pipe section and a fourth pipe section, the third pipe section being connected between an outlet of the second pipe section and the first rotor, and the fourth pipe section being connected between the first stator and the refrigerating device, and the third pipe section and the fourth pipe section being in fluid communication through a second fluid passage between the first stator and the first rotor.

In some embodiments, the scanning inspection device further includes a first slip ring, a first cable and a second cable, wherein the first slip ring includes a second stator and a second rotor rotationally connected to the second stator, the second stator being connected to the first rotating frame, and the second rotor being connected to the second rotating frame; the first cable is connected between the power supply source and the second stator, and the second cable is connected between the second rotor and an electrical socket of the refrigerating device, and electrical connection between the first cable and the second cable is implemented by an electrical connection circuit between the second stator and the second rotor; and an axis of rotation of the rotary joint, an axis of rotation of the first slip ring, and an axis of rotation of the second rotating frame relative to the first rotating frame are collinear.

In some embodiments, the first rotating frame includes a first support plate and a second support plate connected to the first support plate, wherein a preset distance is formed between the first support plate and the second support plate; the second rotating frame is disposed between the first support plate and the second support plate; the rotary joint is disposed between the second rotating frame and the first support plate; and the first slip ring is disposed between the second rotating frame and the second support plate.

In some embodiments, the scanning inspection device further includes a first support body, a second support body, a first slewing bearing, and a second slewing bearing, wherein the first support body is connected to the first support plate, and the first slewing bearing is supported on the first support body and connected to the second rotating frame; and the second support body is connected to the second support plate, and the second slewing bearing is supported on the second support body and connected to the second rotating frame.

In some embodiments, the first support body includes a first hollow cylinder, and the first stator is mounted in the first hollow cylinder; and/or the second support body includes a second hollow cylinder, and the second stator is mounted in the second hollow cylinder.

In some embodiments, an outer ring of the first slewing bearing and/or the second slewing bearing is provided with an outer tooth, and the second drive device includes a gear and a drive unit, the gear being engaged with the outer tooth, and the drive unit being configured to drive the gear to rotate.

In some embodiments, the scanning inspection device further includes a second slip ring, which is connected between the first cable and the power supply source, and an axis of rotation of the second slip ring is collinear with the axis of rotation of the first rotating frame.

In some embodiments, the scanning inspection device further includes a detection device configured to detect a rotation angle of the second rotating frame, and the second drive device is in signal connection with the detection device to adjust the rotation angle of the second rotating frame relative to the first rotating frame based on a detection result from the detection device.

In some embodiments, the detection device includes an encoder.

Based on the above technical solution, in embodiments of the present disclosure, by rotating the first rotating frame to cause the ray source and the detector to rotate relative to the object to be inspected, 360° scanning inspection can be implemented on the object to be inspected, which improves the full coverage and reliability of scanning inspection; moreover, the first rotating frame is also provided with a cooling device, which can cool the ray source in time and prevent failure or damage of the ray source caused by too much heat generated due to working for too long time; and the scanning inspection device also includes a second drive device, which can drive the cooling device to rotate toward a direction opposite to a rotation direction of the first rotating frame to maintain a relatively fixed attitude of the cooling device, and prevent dysfunction or failure of the cooling device due to tilting or inverting in the process of rotating with the first rotating frame, which is conducive to ensuring normal working of the cooling device in the process of rotation.

### Brief Description of the Drawings

Drawings illustrated herein are used for providing further understanding of the present disclosure and form part of the present application, and illustrative embodiments of the present disclosure and description thereof are intended for explaining instead of improperly limiting the present disclosure. In the drawings:
Fig. 1 is a schematic structural diagram of an embodiment of a scanning inspection device of the present disclosure.
Fig. 2 is a changing state diagram of an embodiment of a scanning inspection device of the present disclosure in a rotating process.
Fig. 3 is a side view of an embodiment of a scanning inspection device of the present disclosure.
Fig. 4 is a schematic structural diagram of a second rotating frame and structures cooperating therewith in an embodiment of a scanning inspection device of the present disclosure.
Fig. 5 is a cross-sectional view of a second rotating frame and structures cooperating therewith in an embodiment of a scanning inspection device of the present disclosure.

### Reference numerals:

1, first rotating frame; 1a, first support plate; 1b, second support plate; 2, ray source; 3, detector; 4, refrigerating device; 5, conveying device; 6, object to be inspected; 7, second rotating frame; 8, rotary joint; 81, first stator; 82, first rotor; 9, first conveying pipe; 91, first pipe section; 92, second pipe section; 10, second conveying pipe; 101, third pipe section ; 102, fourth pipe section; 11, first slip ring; 111, second stator; 112, second rotor; 12, connecting cable; 121, first cable; 122, second cable; 13, first support body; 14, second support body; 15, first slewing bearing; 16, second slewing bearing; 161, outer tooth.

### Detailed Description of the Embodiments

Technical solutions in the embodiments will be described below clearly and completely in conjunction with the accompanying drawings in the embodiments of the present disclosure. Obviously, the described embodiments are only part of, instead of all of the embodiments of the present disclosure. Based on the embodiments in the present disclosure, all of other embodiments obtained by those of ordinary skill in the art without creative work should fall into the protection scope of the present disclosure.

In description of the present disclosure, it should be understood that orientation or position relations denoted by the terms "center", "transverse", "longitudinal", "front", "rear", "left", "right", "upper", "lower", "vertical", "horizontal", "top", "bottom", "inner", "outer" and the like are orientation or position relations illustrated based on the drawings, are merely for the convenience of describing the present disclosure and simplifying description, instead of indicating or implying the denoted devices or elements necessarily have specific orientations or be constructed and operated in specific orientations, and thus the terms cannot be understood as limiting the protection scope of the present disclosure.

In order to cool a ray source in a rotating scanning inspection system, the inventors have made various attempts and found that the ray source rotates continuously during inspection, and if the cooling system is disposed in a fixed position, a connecting device between the cooling system and the ray source will be winded and twisted during rotation of the ray source, and a connecting cable will break and affect normal working of the scanning system after long time of rotation; and if the cooling system rotates together with the ray source, the cooling system will be tilted or inverted relative to the ground. For example, the cooling system includes a compressor, and operation fault will occur in the compressor after tilting or inversion. Thus, there are also some problems if the cooling system is simply configured to rotate with the ray source.

After further research, the inventors propose a technical solution that a cooling system is configured to rotate together with a ray source while rotating around its axis relative to the ray source, which can effectively solve the problem of failure caused by tilting or inverting of the cooling system, and at the same time, can also achieve effective cooling of the ray source.

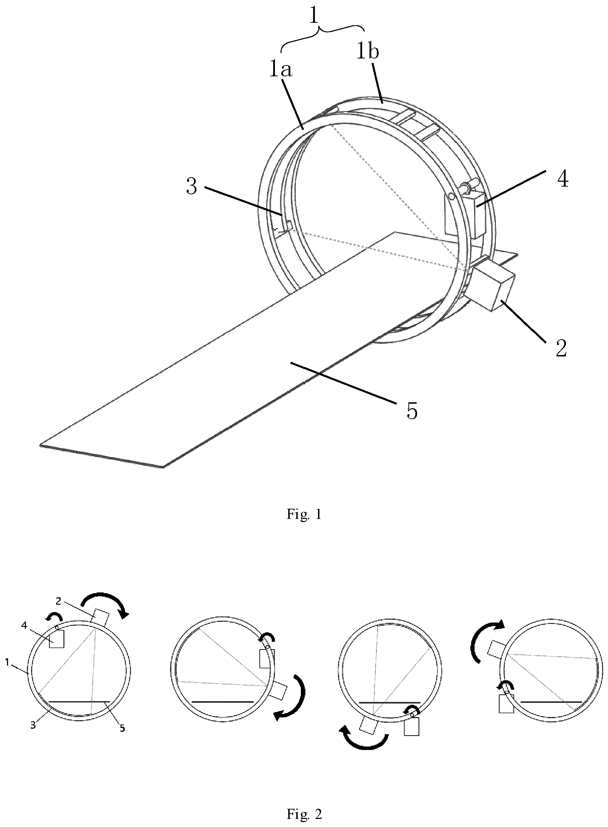

As shown in Figs. 1 to 3, in some embodiments of a scanning inspection device provided in the present disclosure, the scanning inspection device includes a first rotating frame 1, a first drive device, a ray source 2, a detector 3, a second drive device, and a cooling device for cooling the ray source 2. A conveying channel for passage of an object to be inspected 6 is provided in the center of the first rotating frame 1. The first drive device is in drive connection with the first rotating frame 1 to drive the first rotating frame 1 to rotate. The ray source 2, the detector 3, and the cooling device for cooling the ray source 2 are each mounted on the first rotating frame 1 and rotate with the first rotating frame 1. The second drive device is configured to drive the cooling device to rotate toward a direction opposite to a rotation direction of the first rotating frame 1, so that the cooling device is maintained in a fixed attitude during rotation with the first rotating frame 1.

In the above embodiments, by rotating the first rotating frame 1 to cause the ray source 2 and the detector 3 to rotate relative to the object to be inspected 6, 360° scanning inspection can be implemented on the object to be inspected 6, which improves the full coverage and reliability of scanning inspection; moreover, the first rotating frame 1 is also provided with a cooling device, which can cool the ray source in time and prevent failure or damage of the ray source caused by too much heat generated due to working for too long time; and the scanning inspection device also includes a second drive device, which can drive the cooling device to rotate toward a direction opposite to a rotation direction of the first rotating frame 1 to maintain a relatively fixed attitude of the cooling device, prevent the cooling device from tilting or inverting in the process of rotating with the first rotating frame 1, and ensure normal working of the cooling device in the process of rotation.

In some embodiments, the first drive device drives the first rotating frame 1 to rotate relative to the ground, and the ray source 2, the detector 3, and the cooling device revolve with the first rotating frame 1, and at the same time the cooling device also rotates around its axis relative to the first rotating frame 1. The rotation direction of the cooling device around its axis is opposite to its revolution direction, to keep the cooling device relatively fixed with respect to the ground, such as keeping the cooling device vertical at all the time during the revolution, prevent the cooling device from tilting or inverting, and ensure normal working of the cooling device.

As shown in Fig. 2, during rotation of the first rotating frame 1, the cooling device simultaneously rotates around its axis in the opposite direction, and the cooling device always maintains a relatively fixed attitude with respect to the ground without tilting.

In some embodiments, the ray source 2 and the detector 3 are spaced apart in a circumferential direction of the first rotating frame 1, and a ray emitted by the ray source 2 is irradiated on the object to be inspected 6 located in the conveying channel, and the ray is received by the detector 3 after passing through the object to be inspected 6, thereby achieving scanning inspection.

In some embodiments, a conveying device 5 is provided in the conveying channel, and the conveying device 5 is configured to convey the object to be inspected 6 to pass through the conveying channel, thereby accomplishing scanning inspection during the conveying process. The conveying device 5 may be a conveyor belt or a conveyor chain, or the like.

In some embodiments, the cooling device is disposed outside a scanning range of the ray source 2. Such configuration can avoid the cooling device's influence on the scanning inspection of the object to be inspected 6 and improve the accuracy of the scanning inspection.

In some embodiments, the scanning inspection device further includes a second rotating frame 7. The second rotating frame 7 is mounted on the first rotating frame 1, the cooling device is mounted on the second rotating frame 7, and the second drive device is in drive connection with the second rotating frame 7 to drive the second rotating frame 7 to rotate relative to the first rotating frame 1.

By providing the second rotating frame 7, the cooling device can be caused to rotate around its axis by the rotation of the second rotating frame 7, the installation of the cooling device is facilitated, and direct connection between the cooling device and the second drive device can also be avoided, which is conducive to protecting the cooling device.

In some embodiments, the cooling device includes a refrigerating device 4 and a first conveying pipe 9. The first conveying pipe 9 is in communication with the refrigerating device 4 and configured to convey a heat exchange medium from the refrigerating device 4 to the ray source 2 to cool the ray source. A portion of the heat exchange medium is taken from the refrigeration device 4 to cool the ray source 2, and the heat exchange medium may absorb heat emitted by the ray source 2, to avoid damaging the ray source itself or neighboring components by the heat emitted by the ray source 2, and improve the service life of the ray source 2 and the neighboring components.

In some embodiments, the cooling device further includes a second conveying pipe 10. The second conveying pipe 10 is connected between an outlet of the first conveying pipe 9 and the refrigerating device 4 to return the heat exchange medium after heat exchange with the ray source 2 to the refrigerating device 4.

By providing the second conveying pipe 10, the cooling device 4, the first conveying pipe 9 and the second conveying pipe 10 can form a closed-loop circuit to achieve continuous cooling of the ray source 2, and it can also simplify the structure of the cooling device, and reduce connections between the cooling device, and other components that do not rotate with the first rotating frame 1.

In some embodiments, the first conveying pipe 9 is wound around an outer periphery of the ray source 2, and the heat exchange medium in the first conveying pipe 9 exchanges heat with the ray source 2; or the first conveying pipe 9 is communicated with a cooling flow path inside the ray source 2, and the first conveying pipe 9 conveys the heat exchange medium to the interior of the ray source 2 to cool the ray source 2.

In some embodiments, the refrigerating device 4 includes a compressor, a condenser, and a throttling element, and a heat exchange section of the first conveying pipe 9 with the ray source 2 may act as an evaporator of the refrigerating device 4 to implement a refrigeration cycle. The heat exchange medium conveyed by the first conveying pipe 9 is a refrigerant.

In some embodiments, the refrigerating device 4 includes a compressor, a condenser, a throttling element, and an evaporator. The heat exchange medium conveyed by the first conveying pipe 9 undergoes heat exchange with the evaporator in the refrigerating device 4, and the cooled heat exchange medium is conveyed to the ray source 2 to cool the ray source 2. The heat exchange medium may be water, or the like.

Since the first conveying pipe 9 is provided between the cooling device and the ray source 2, the ray source 2 rotates with the first rotating frame 1, and the cooling device rotates around itself while revolving, a rotary joint is used for connections in order to avoid winding and twisting of the first conveying pipe 9.

In some embodiments, the scanning inspection device further includes a rotary joint 8. The rotary joint 8 includes a first stator 81 and a first rotor 82 rotationally connected to the first stator 81. The first stator 81 is connected to the first rotating frame 1, and the first rotor 82 is connected to the second rotating frame 7. The first conveying pipe 9 includes a first pipe section 91 and a second pipe section 92. The first pipe section 91 is connected between the refrigeration device 4 and the first stator 81, and the second pipe section 92 is connected to the first rotor 82. The first pipe section 91 and the second pipe section 92 are in fluid communication through a first fluid passage between the first stator 81 and the first rotor 82.

By providing the rotary joint 8, the second pipe section 92 can rotate relative to the first pipe section 91, which, on the basis of implementing conveying of the heat exchange medium, can also avoid winding of the first conveying pipe 9 due to rotation of the cooling device around its axis.

For embodiments in which the scanning inspection device further includes a second conveying pipe 10, the scanning inspection device further includes a rotary joint 8. The rotary joint 8 includes a first stator 81 and a first rotor 82 rotationally connected to the first stator 81. The first stator 81 is connected to the first rotating frame 1, and the first rotor 82 is connected to the second rotating frame 7. The first conveying pipe 9 includes a first pipe section 91 and a second pipe section 92. The first pipe section 91 is connected between the refrigeration device 4 and the first stator 81, and the second pipe section 92 is connected to the first rotor 82. The first pipe section 91 and the second pipe section 92 are in fluid communication through a first fluid passage between the first stator 81 and the first rotor 82. The second conveying pipe 10 includes a third pipe section 101 and a fourth pipe section 102. The third pipe section 101 is connected between an outlet of the second pipe section 92 and the first rotor 82, and the fourth pipe section 102 is connected between the first stator 81 and the refrigerating device 4. The third pipe section 101 and the fourth pipe section 102 are in fluid communication through a second fluid passage between the first stator 81 and the first rotor 82.

By using the rotary joint 8 having the first fluid passage and the second fluid passage, winding of the first conveying pipe 9 and the second conveying pipe 10 can be avoided at the same time.

In some embodiments, the number of the first conveying pipe 9 is two or more, and the number of the second conveying pipe 10 may also be two or more.

For a cooling device that needs to be connected to a power supply source, it also needs to solve the problem of winding of a connecting cable.

In some embodiments, the scanning inspection device further includes a first slip ring 11 and a connecting cable 12. The connecting cable 12 includes a first cable 121 and a second cable 122. The first slip ring 11 includes a second stator 111 and a second rotor 112 rotationally connected to the second stator 111. The second stator 111 is connected to the first rotating frame 1, and the second rotor 112 is connected to the second rotating frame 7. The first cable 121 is connected between the power supply source and the second stator 111, and the second cable 122 is connected between the second rotor 112 and an electrical socket of the refrigerating device 4. Electrical connection between the first cable 121 and the second cable 122 is implemented by an electrical connection circuit between the second stator 111 and the second rotor 112. An axis of rotation of the rotary joint 8, an axis of rotation of the first slip ring 11, and an axis of rotation of the second rotating frame 7 relative to the first rotating frame 1 are collinear.

By providing the first slip ring 11, the second cable 122 may rotate relative to the first cable 121, to avoid the problem of winding of the connecting cable caused during rotation of the cooling device around its axis.

By setting the axis of rotation of the rotary joint 8, the axis of rotation of the first slip ring 11, and the axis of rotation of the second rotating frame 7 relative to the first rotating frame 1 to be collinear, it can avoid winding of the first conveying pipe 9, the second conveying pipe 10, and the connecting cable 12 while the cooling device rotates around its axis.

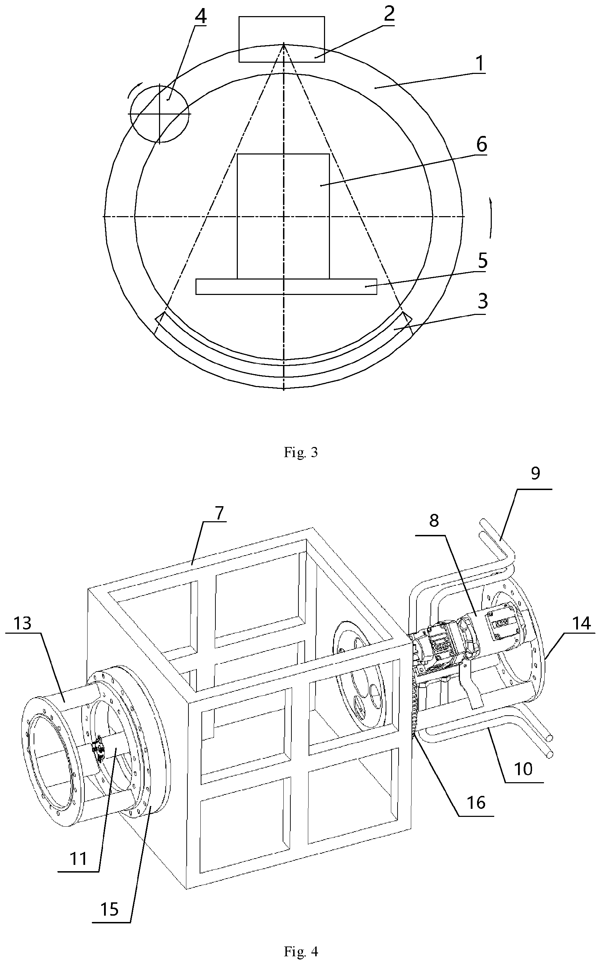

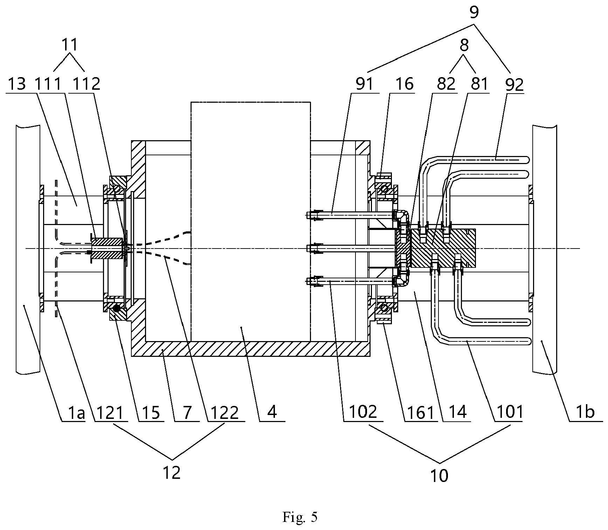

As shown in Figs. 4 and 5, in some embodiments, the first rotating frame 1 includes a first support plate 1a and a second support plate 1b connected to the first support plate 1a. A preset distance is formed between the first support plate 1a and the second support plate 1b. The second rotating frame 7 is disposed between the first support plate 1a and the second support plate 1b. The rotary joint 8 is disposed between the second rotating frame 7 and the first support plate 1a. The first slip ring 11 is disposed between the second rotating frame 7 and the second support plate 1b. Such an arrangement facilitates achieving collinearity of the axis of rotation of the rotary joint 8, the axis of rotation of the first slip ring 11, and the axis of rotation of the second rotating frame 7 relative to the first rotating frame 1, and can also achieve relative independence of the heat exchange medium and the electrical connection, and avoid the intersection and mutual influence of the first conveying pipe 9, the second conveying pipe 10, and the connecting cable 12.

In some embodiments, the scanning inspection device further includes a first support body 13, a second support body 14, a first slewing bearing 15 and a second slewing bearing 16. The first support body 13 is connected to the first support plate 1a, and the first slewing bearing 15 is supported on the first support body 13 and connected to the second rotating frame 7. The second support body 14 is connected to the second support plate 1b, and the second slewing bearing 16 is supported on the second support body 14 and connected to the second rotating frame 7.

By providing the first support body 13, the second support body 14, the first slewing bearing 15 and the second slewing bearing 16, the second rotating frame 7 can be supported, and sufficient space can also be retained for the arrangement of the rotary joint 8, the first slip ring 11, the first conveying pipe 9, the second conveying pipe 10 and the connecting cable 12.

In some embodiments, the first support body 13 includes a first hollow cylinder, and the first stator 81 is mounted in the first hollow cylinder; and/or the second support body 14 includes a second hollow cylinder, and the second stator 111 is mounted in the second hollow cylinder.

The first support body 13 and the second support body 14 each include a hollow cylinder, which can reduce the weight of the first support body 13 and the second support body 14, while facilitating the arrangement of the rotary joint 8 and the first slip ring 11.

In some embodiments, a first positioning plate is provided in the first hollow cylinder, and the first stator 81 is mounted on the first positioning plate; and a second positioning plate is provided in the second hollow cylinder, and the second stator 111 is mounted on the second positioning plate.

In some embodiments, a third positioning plate is provided on one side of the second rotating frame 7, and the first rotor 82 is mounted on the third positioning plate; and a fourth positioning plate is provided on the other side of the second rotating frame 7, and the second rotor 112 is mounted on the fourth positioning plate.

In some embodiments, an outer ring of the first slewing bearing 15 and/or the second slewing bearing 16 is provided with an outer tooth 161, and the second drive device includes a gear and a drive unit, the gear being engaged with the outer tooth 161, and the drive unit being configured to drive the gear to rotate.

The second drive device drives the first slewing bearing 15 and/or the second slewing bearing 16 to rotate by transmission between the gear and the outer tooth 161.

In some embodiments, the scanning inspection device further includes a second slip ring. The second slip ring is connected between the first cable 121 and the power supply source, and an axis of rotation of the second slip ring is collinear with the axis of rotation of the first rotating frame 1.

By providing the second slip ring, the first cable 121 can be rotated relative to the power supply source to prevent winding of the first cable 121 during rotation of the first rotating frame 1.

The power supply source may be power supply equipment disposed on the ground, and may also be a utility power socket.

In some embodiments, the scanning inspection device may further include a power supply battery, which is mounted on the first rotating frame 1, such that the first cable 121 is maintained fixed relative to the power supply battery, and the second slip ring may be omitted.

In some embodiments, the scanning inspection device further includes a detection device configured to detect a rotation angle of the second rotating frame 7. The second drive device is in signal connection with the detection device to adjust the rotation angle of the second rotating frame 7 relative to the first rotating frame 1 based on a detection result from the detection device.

By providing the detection device, the rotation angle of the second rotating frame 7 can be detected in real time, and the second drive device controls the rotation angle of the second rotating frame 7 according to the detection result from the detection device, which is conducive to keeping the second rotating frame 7 relatively fixed with respect to the ground, thereby maintaining a fixed attitude of the cooling device with respect to the ground, and preventing the cooling device from tilting or inverting in the process of revolution.

In some embodiments, the detection device includes an encoder. The encoder has a simple structure, and detection results are reliable.

In the above embodiments, the first drive device and the second drive device may be motors or other drive devices. The second drive device may be mounted on the first rotating frame 1 to drive the second rotating frame 7 to rotate relative to the first rotating frame 1.

In some embodiments, the first rotating frame 1 is in a circular ring shape.

In some embodiments, the second rotating frame 7 is in a cube or cuboid shape, which is conducive to ensuring the steadiness of the cooling device.

Finally, it should be noted that the above embodiments are only used for describing rather than limiting the technical solutions of the present disclosure. Although the present disclosure is described in detail with reference to the preferred embodiments, those of ordinary skill in the art should understand that without departing from the principles of the disclosure, they still can make modifications to the specific implementations in the present disclosure or make equivalent substitutions to part of technical features thereof; and such modifications and equivalent substitutions should be encompassed within the technical solutions sought for protection in the present disclosure.

## Claims

1. A scanning inspection device, comprising:
a first rotating frame (1), with a conveying channel for passage of an object to be inspected (6) being provided in the center of the first rotating frame (1);
a first drive device in drive connection with the first rotating frame (1) to drive the first rotating frame (1) to rotate,
wherein a ray source (2), a detector (3), and a cooling device for cooling the ray source (2) are each mounted on the first rotating frame (1) and rotate with the first rotating frame (1); and
a second drive device configured to drive the cooling device to rotate toward a direction opposite to a rotation direction of the first rotating frame (1), so that the cooling device is maintained in a fixed attitude during rotation with the first rotating frame (1).

2. The scanning inspection device according to claim 1, wherein the cooling device is disposed outside a scanning range of the ray source (2).

3. The scanning inspection device according to claim 1 or 2, further comprising a second rotating frame (7), the second rotating frame (7) being mounted on the first rotating frame (1), the cooling device being mounted on the second rotating frame (7), and the second drive device being in drive connection with the second rotating frame (7) to drive the second rotating frame (7) to rotate relative to the first rotating frame (1).

4. The scanning inspection device according to claim 3, wherein the cooling device comprises a refrigerating device (4) and a first conveying pipe (9), the first conveying pipe (9) being in communication with the refrigerating device (4) and configured to convey a heat exchange medium from the refrigerating device (4) to the ray source (2) for cooling thereof.

5. The scanning inspection device according to claim 4, wherein the cooling device further comprises a second conveying pipe (10), the second conveying pipe (10) being connected between an outlet of the first conveying pipe (9) and the refrigerating device (4) to return the heat exchange medium after heat exchange with the ray source (2) to the refrigerating device (4).

6. The scanning inspection device according to claim 4 or 5, further comprising a rotary joint (8), the rotary joint (8) comprises a first stator (81) and a first rotor (82) rotationally connected to the first stator (81), the first stator (81) being connected to the first rotating frame (1), and the first rotor (82) being connected to the second rotating frame (7); and the first conveying pipe (9) comprises a first pipe section (91) and a second pipe section (92), the first pipe section (91) being connected between the refrigeration device (4) and the first stator (81), and the second pipe section (92) being connected to the first rotor (82), and the first pipe section (91) and the second pipe section (92) being in fluid communication through a first fluid passage between the first stator (81) and the first rotor (82).

7. The scanning inspection device according to claim 5, further comprising a rotary joint (8), which comprises a first stator (81) and a first rotor (82) rotationally connected to the first stator (81), the first stator (81) being connected to the first rotating frame (1), and the first rotor (82) being connected to the second rotating frame (7); the first conveying pipe (9) comprises a first pipe section (91) and a second pipe section (92), the first pipe section (91) being connected between the refrigeration device (4) and the first stator (81), and the second pipe section (92) being connected to the first rotor (82), and the first pipe section (91) and the second pipe section (92) being in fluid communication through a first fluid passage between the first stator (81) and the first rotor (82); and the second conveying pipe (10) comprises a third pipe section (101) and a fourth pipe section (102), the third pipe section (101) being connected between an outlet of the second pipe section (92) and the first rotor (82), and the fourth pipe section (102) being connected between the first stator (81) and the refrigerating device (4), and the third pipe section (101) and the fourth pipe section (102) being in fluid communication through a second fluid passage between the first stator (81) and the first rotor (82).

8. The scanning inspection device according to claim 6 or 7, further comprising a first slip ring (11), a first cable (121) and a second cable (122), wherein the first slip ring (11) comprises a second stator (111) and a second rotor (112) rotationally connected to the second stator (111), the second stator (111) being connected to the first rotating frame (1), and the second rotor (112) being connected to the second rotating frame (7); the first cable (121) is connected between the power supply source and the second stator (111), and the second cable (122) is connected between the second rotor (112) and an electrical socket of the refrigerating device (4), and electrical connection between the first cable (121) and the second cable (122) is implemented by an electrical connection circuit between the second stator (111) and the second rotor (112); and an axis of rotation of the rotary joint (8), an axis of rotation of the first slip ring (11), and an axis of rotation of the second rotating frame (7) relative to the first rotating frame (1) are collinear.

9. The scanning inspection device according to claim 8, wherein the first rotating frame (1) comprises a first support plate (1a) and a second support plate (1b) connected to the first support plate (1a), wherein a preset distance is formed between the first support plate (1a) and the second support plate (1b); the second rotating frame (7) is disposed between the first support plate (1a) and the second support plate (1b); the rotary joint (8) is disposed between the second rotating frame (7) and the first support plate (1a); and the first slip ring (11) is disposed between the second rotating frame (7) and the second support plate (1b).

10. The scanning inspection device according to claim 9, further comprising a first support body (13), a second support body (14), a first slewing bearing (15), and a second slewing bearing (16), wherein the first support body (13) is connected to the first support plate (1a), and the first slewing bearing (15) is supported on the first support body (13) and connected to the second rotating frame (7); and the second support body (14) is connected to the second support plate (1b), and the second slewing bearing (16) is supported on the second support body (14) and connected to the second rotating frame (7).

11. The scanning inspection device according to claim 10, wherein the first support body (13) comprises a first hollow cylinder, and the first stator (81) is mounted in the first hollow cylinder; and/or the second support body (14) comprises a second hollow cylinder, and the second stator (111) is mounted in the second hollow cylinder.

12. The scanning inspection device according to claim 10 or 11, wherein an outer ring of the first slewing bearing (15) and/or the second slewing bearing (16) is provided with an outer tooth (161), and the second drive device comprises a gear and a drive unit, the gear being engaged with the outer tooth (161), and the drive unit being configured to drive the gear to rotate.

13. The scanning inspection device according to any one of claims 8-12, further comprising a second slip ring, the second slip ring is connected between the first cable (121) and the power supply source, and an axis of rotation of the second slip ring is collinear with the axis of rotation of the first rotating frame (1).

14. The scanning inspection device according to any one of claims 3-13, further comprising a detection device configured to detect a rotation angle of the second rotating frame (7), and the second drive device is in signal connection with the detection device to adjust the rotation angle of the second rotating frame (7) relative to the first rotating frame (1) based on a detection result from the detection device.

15. The scanning inspection device according to claim 14, wherein the detection device comprises an encoder.
